# EUROPEAN PATENT SPECIFICATION

(11) **EP 0 869 742 B1**
(45) Date of publication and mention of the grant of the patent: **21.05.2003**
(21) Application number: 96941759.1
(22) Date of filing: 11.12.1996
(51) Int. Cl.: A61B 18/12

(54) **AN ELECTROSURGICAL INSTRUMENT AND AN ELECTROSURGICAL ELECTRODE ASSEMBLY**
ELEKTROCHIRURGISCHES GERÄT UND ELEKTRODENVORRICHTUNG
INSTRUMENT ELECTROCHIRURGICAL ET ENSEMBLE ELECTRODE ELECTROCHIRURGICAL

(30) Priority: 29.12.1995 GB 9526627; 03.05.1996 GB 9609280
(43) Date of publication of application: 14.10.1998
(73) Proprietor: Gyrus Medical Limited, St. Mellons, Cardiff CF3 0LT (GB)
(72) Inventor: GOBLE, Nigel Mark, Near Cardiff CF3 8SB (GB); GOBLE, Colin Charles Owen, Penarth, South Glamorgan CF64 1AT (GB)
(74) Representative: Blatchford, William Michael
(86) International application number: GB9603044
(87) International publication number: WO97024073

(56) References cited:
- WO-A-94/10921
- DE-A- 3 427 517
- DE-A- 4 339 049

## Description

This invention relates to an electrosurgical instrument having a generator for generating radio frequency power, a handpiece, and a detachable electrode assembly. The invention also includes an electrode assembly for detachable mounting to a handpiece of an electrosurgical instrument, a method of assembling and operating the instrument, and an electrosurgical generator.

Surgery by the application of radio frequency currents to living tissue to desiccate, cut, or vaporise the tissue using one or more electrodes coupled to a radio frequency generator raises particular problems in terms of obtaining efficient transfer of power from the generator to the tissue and producing a required surgical effect in a controllable manner. The electrical characteristics of an electrode assembly when in use can vary widely depending on the mode of use, the conductivity of the tissue and surrounding substances, and the nature of the assembly itself.

These problems are particularly evident in the case of electrosurgery performed with an electrode or electrodes immersed in liquid at the operation site (often referred to as "underwater" electrosurgery).

Underwater surgery is commonly performed using endoscopic techniques in which (1) the endoscope itself may provide a conduit for passage of an electrode, commonly referred to as a working channel, or (2) the endoscope may be specifically adapted to include means for mounting an electrode, such as are provided on a resectoscope, or (3) the electrode may be introduced to the body cavity via separate access means at an angle with respect to the endoscope; a technique commonly referred to as triangulation. These variations in technique can be subdivided by surgical speciality, where one or other of the techniques has particular advantages given the access route to the specific body cavity. Endoscopes with integral working channels or those characterised as resectoscopes, are generally employed when access to the body cavity may be through a natural body opening; such as the cervical canal to gain access to the endometrial cavity of the uterus or the urethra to gain access to the prostate gland and bladder. Endoscopes specifically designed for use in the endometrial cavity are referred to as hysteroscopes. Those for the urinary tract include cystoscopes. urethroscopes and resectoscopes used during transurethral resection or vaporisation of the prostate gland (TURP and EVAP), respectively). When there is no natural body opening through which the endoscope may be passed, the technique of triangulation is commonly employed. A common site where triangulation is used is during underwater endoscopic surgery on joint cavities such as the knee and shoulder. The endoscope used in these procedures is commonly referred to as an arthroscope.

The present invention may be applied in an electrosurgical generator and an electrode assembly designed for operation with electrodes immersed in a conductive liquid such as saline solution. The electrode assembly has two electrodes; a first, active electrode at the extreme distal end of the assembly for contacting the tissue to be treated, and a second, return electrode spaced proximally from the active electrode and separated from the latter by an insulation barrier. When the electrodes are immersed in the saline solution, the solution provides a conductive path between the tissue next to the active electrode and the proximal return electrode which remains spaced from the tissue. The electrode assembly is fed by a generator which includes a rapid-acting power reduction circuit operating to prevent significant vapour formation at the active electrode during electrosurgical desiccation. The output power supplied to the electrode assembly by the generator is rapidly reduced when the peak output voltage reaches a preset threshold with the object of avoiding a rapid runaway increase in power delivery and arcing when vaporisation commences, which would lead to uncontrollable tissue disruption in place of the required desiccation. This effect is especially problematical when the generator has a significant output impedance.

Different electrode assemblies can be used to perform different electrosurgical functions.

DE-A-4339049 refers in general terms to encoding devices in surgical instruments, including the use of different capacitors and tuned circuits in different instruments, as well as programmable encoding devices. Specific circuit details are not disclosed.

DE-A-3427517 discloses the use of different so-called frequency-selective elements housed in the plugs of different comminution or high frequency surgical probes which may be plugged into a generator. The generator includes suitable sensing circuitry for distinguishing the probes by detecting the characteristic frequencies of the frequency-selective elements. Isolation of the circuitry is performed by a plurality of optoelectronic isolation devices.

It is an object of the present invention to provide an electrosurgical instrument which produces a controllable surgical effect and which is versatile in use.

According to this invention there is a provided an electrosurgical instrument comprising a first unit including a generator for generating radio frequency power, and a second unit including an electrode assembly, the second unit being detachably connectible to the first unit such that radio frequency power can be conveyed to the electrode assembly, characterised in that the second unit includes an identification capacitor having a value indicative of a characteristic of the electrode assembly, and the first unit includes sensing means for sensing the value of the capacitor when the second unit is connected to the first unit. the generator including adjustment means responsive to the sensing means to adjust the output of the generator so as to suit the indicated electrode assembly characteristic, and characterised in that the sensing means includes an inductance which forms a resonant circuit with the identification capacitor when the second unit is connected to the first unit, the resonant frequency of the resonant circuitry being dependent on the value of the capacitor. In this way the generator can be configured automatically to suit a variety of different electrode assemblies so that the same generator can be used for different electrosurgical operations. the operator being relieved of much of the task of setting the generator to suit the selected electrode assembly.

This is particularly useful in the case of so-called underwater electrosurgery as described above and in the above-mentioned co-pending application. The applicants have found that the power levels which can be applied whilst performing desiccation vary widely depending on the construction of the electrode assembly. By including in each electrode assembly an identification capacitor which is indicative of, for example, a vaporisation power threshold of the assembly, this characteristic of the assembly can be communicated to the generator so that the radio frequency output can be set accordingly. Thus, improved control, particularly in desiccation operations, can be achieved whilst maintaining the ability to use different electrode assemblies with the same generator.

For cutting and tissue vaporisation. vaporisation of the immersion liquid is required, but the power level applied should not exceed that which causes damage to the electrode assembly. It is possible to use the present invention to set a radio frequency voltage limit so as to limit the extent of tissue vaporisation to avoid exceeding the power rating of the electrode assembly.

The first unit may comprise the generator, a connector (which may be integrated into an instrument handpiece), and a cable for coupling the generator to the connector. In this case the second unit may be constituted by the electrode assembly, which assembly itself has a connector which mates with the connectors of the first unit, thereby providing a demountable mechanical interface.

Alternatively, the first unit may comprise a generator with a first connector, while the second unit comprises the combination of the electrode assembly, a second connector, and a cable connecting the second connector with the electrode assembly, so that the mechanical interface is provided on the generator. In this case, the electrode assembly may be detachably or non-detachably mounted in a handpiece or a housing forming part of the second unit.

In both configurations, the mechanical interface may provide further means for controlling or adjusting the generator according to the component connected to it. In particular, the interface may comprise a specific plug and socket combination in which both the plug and the socket are shaped such that neither can be used with a different socket or plug respectively. Consequently different plug and socket combinations can be provided for different surgical applications. For instance, a first combination may be used for hysteroscopic system, another one for arthroscopic applications, and so on.

Accordingly, if the generator has a socket of a type which is designated for, say, hysteroscopic procedures, the generator circuitry is arranged to supply output signals to that socket which are suited to such procedures. If, on the other hand, the generator has a socket of a type designated for arthroscopic procedures, the generator circuitry supplies signals to that socket which are more suited to arthroscopic procedures. A range of electrode assemblies may be provided for each class of procedures, and the generator can be configured such that the electrical identification components in the electrode assemblies for one class of procedures affects the generator output differently from the same group of identification components when used in electrode assemblies for a different class of procedures. In effect, the generator is configured to provide signals which are adjustable in response to the identification component according to the type of socket to which the output signals are supplied. In this way, the available range of different signal outputs is expanded beyond that available by simply adjusting the output according to the value of the identification component.

It is in this light that the preferred instrument in accordance with the invention provides a mechanical interface between the first and second units which is formed of a plug and a socket having respective interfitting configurations selected from a range of different shapes so that the first and second units are operable together only if they have such interfitting plug and socket shapes.

More specifically, in a preferred electrosurgical instrument in accordance with the invention, the first unit includes an output connector for delivering output signals to the second unit, the configuration of the output connector being specific to the field of the surgery for which the output signals are suited. The second unit has an input connector for receiving the output signals from the generator and supplying them to the electrode assembly, the configuration of the input connector being specific to the field of surgery for which the electrode assembly is suited. The configurations of the input and the output connectors form an interfitting combination such that the electrode assembly and the generator are interoperable only when both the electrode assembly and the output signals applied to the said connector are suited to the same field of surgery.

The manner in which the sensing means and the adjustment means respond to the identification component is dependent on the configuration of the said output connector.

As a result, the electrode configurations and the respective electrosurgical output can be optimised depending on the type of endoscope, and hence the type of surgical procedure, being used. The system, comprising specific electrode assemblies, the generator, and connector means between the electrosurgical generator and a selected electrode assembly, can be categorised as an arthrascopic system, a hysteroscopic system or an endoscopic urological system, using a unique cable/plug assembly for each of the speciality segments.

The arthroscopic electrodes may be characterised as short (100 - 140mm), rigid with a working diameter up to 4mm. They are introduced through a stab incision into the joint cavity with or without a cannula using the triangulation technique. The tissue to be treated is commonly dense and of high electrical impedance, such as meniscal cartilage. Output power and voltage settings reflect both the type of tissue, the size of electrode and the fact that arthroscopists are seeking a speed of effect comparable to the mechanical shaver devices they current employ, albeit from an electrode of smaller diameter than shaver blades to improve access. Arthroscopic electrode assembly designs therefore need to support relatively high output specifications, produce rapid debulking of high impedance tissue and must connect to an ergonomic handpiece to aid tissue manipulation. In the arthroscopic system, then, the range of electrode assembly identifications are subdivided to arthroscopic electrode assembly identification and generator system set-up settings according to these specifications and surgical techniques.

The hysteroscopic electrodes may be characterised as long (350 - 400mm), flexible or semi-rigid, and with a working diameter typically in the range of 1.27 - 2.86mm (4-9 Fr). They are introduced through a working channel. The tissue is commonly more vascular than that encountered during arthroscopic surgery and inadvertent perforation of the uterus represents a serious complication. It is desirable therefore to support a more controlled application using electrodes with good desiccation capability using more precise movement of the electrode or hysteroscope than is normal during arthroscopic procedures. The electrode assembly to generator interface does not require a true "handpiece" and may merely constitute connector means. In the hysteroscopic system, the range of electrode identifications are, therefore, differently subdivided to hysteroscopic electrode identification and generator system set-up settings according to these specifications and surgical techniques.

There are two main electrode configurations for endoscopic urological procedures: (1), cystoscopic/urethroscopic electrode, and; (2), resectoscope electrodes. The former have characteristics very similar to the hysteroscopic electrode, being introduced through the working channel of urological endoscopes. Resectoscope electrodes are introduced very differently, in that they are mounted on an endoscope prior to passage of the assembled instrument through a working sheath introduced via the urethra. The proximal end of the electrode is connected to a trigger assembly and electrical contact integral to the resectoscope. By this means, the electrode can be moved back and forth through a defined range of motion by operating the trigger mechanism. As the electrode is assembled prior to introduction, the size of the tip is not constrained by working channel dimensions but rather by the working sheath. The working sheath diameter can range up to 10mm. Part of this diameter is occupied by the support wires to the electrode which are commonly bent in a downward angle, with respect to the endoscopic image, to the working tip so that they do not interfere with either visualisation or its operation. Nonetheless, a typical roller electrode may be in the range of 3 - 4mm wide and 2 - 3 mm in diameter. This size is necessary given that, on average, 20 - 30 grams of prostate tissue must be removed. The tissue is of variable consistency as a mixture of fibrous and glandular elements, areas of which may be quite vascular. A combination effect of simultaneous desiccation and vaporisation is, therefore, required. The roller form of urological electrode requires a high power high voltage generator output. In the urological system, the range of electrode identifications are, therefore, subdivided to a further group of urological endoscopic electrode assembly identification and system set-up settings according to these specifications and surgical techniques.

In addition to segmentation of electrode assembly identification to the above underwater surgical specialities, further subdivision of generator settings may be included for electrode assemblies designed for operation in a saline working environment confined to the tip of the electrode. As such, this electrode assembly design significantly increases the system versatility in surgical procedures either performed in open air or under a gaseous distension. The former may be performed under direct vision to debulk, incise or coagulate a tissue mass or by a triangulated endoscopic approach such as those performed to remove spinal disc herniations or to remove diseased tissue from the sinuses; so-called functional endoscopic sinus surgery. Examples of gaseous distension techniques include laparoscopic and gastrointestinal endoscopic surgery. The range of electrode identifications are, therefore, subdivided to yet a further group of saline assisted electrode identification and system set-up settings according to these specifications and surgical techniques.

The electrode assembly preferably comprises at least one electrode, mounting means for detachably mounting the assembly to another part of the instrument; and a plurality of contacts for mating with contacts in the other part of the instrument. The plurality of contacts may include a pair of contacts for conveying radio frequency electrosurgical currents between the electrode assembly and the said other part. The identification capacitor is indicative of the assembly and arranged to form a resonant circuit with an inductance in the sensing circuit associated with the said other part of the instrument, which sensing circuit includes an oscillator arranged to oscillate at the resonant frequency of the resonant circuit, the identification capacitor being connected between one of the contacts for conveying radio frequency electrosurgical currents and a third contact on the assembly.

The identification capacitor value varies from electrode assembly to electrode assembly in the range of, typically, 15pF to 1µF according to a power level threshold for the assembly. In the case of an electrode assembly intended for use in an immersing liquid, the threshold may be that at which vaporisation normally occurs, with the capacitance values preferably increasing with increasing power threshold value.

In the preferred bipolar instrument there are typically three contacts, two for the conduction of radio frequency currents and one for identification of the electrode assembly. A single identification contact arrangement allows identification of three or more different electrode assemblies, depending on the number of different identification parameter values used in the system and which the generator sensing means is capable of distinguishing.

In the above variations on the invention from the complete instrument aspect have been described. Other aspects of the invention are now introduced. It will be understood that the above-described variations apply similarly.

With respect to the generator, according to the invention, an electrosurgical generator for use with a plurality of different electrode assemblies including respective identification capacitors having different values indicative of the characteristics of the electrode assemblies, the generator including sensing means responsive to the capacitor values and means for automatically setting the output of the generator according to the indicated characteristic of a selected electrode assembly connected to the generator, characterised in that the sensing means includes an inductance arranged to form a resonant circuit with the capacitor of each selected electrode assembly, the resonant frequency of which resonant circuit is dependent on the respective capacitor value, and the sensing means are operable to produce an electrical output signal for feeding to the setting means, the nature of which output signal is dependent on the resonant frequency and, therefore, on the value of the capacitor of the selected electrode assembly.

The sensing means may include an oscillating device, for example a suitably connected transistor, with the resonant circuit forming part of a self-oscillating circuit so that the oscillator frequency is determined as the resonant frequency of the resonant circuit. In this case, the oscillator frequency is indicative of the electrode assembly characteristic.

Alternatively, the sensing means may include a variable internal inductance, with means for switching in different inductance values, or for varying the inductance value until a combination which is resonant at a predetermined fixed frequency is found.

The inductance in the sensing means may comprise a first winding of an isolation transformer, this winding being connected between a pair of contacts in a connector or an instrument handpiece which engage contacts on the electrode assembly bridged by the identification capacitor so as to form the resonant circuit when the electrode assembly is connected to the generator. A second winding of the transformer preferably forms part of the oscillator, typically in the form of a sensing winding for providing feedback to the oscillator device. The transformer may have a third, excitation winding coupled to the output of the oscillating device. Alternatively, a second transformer may be provided, this having a first winding coupled to the output of the oscillating device to act as an excitation winding, and a second winding coupled in series in the resonant circuit formed when the electrode assembly is connected to the generator. In this case, two transformer windings, one from each transformer. constitute the inductance which resonates with the identification capacitor in the electrode assembly.

In an electrode assembly having an active electrode and a return electrode as described above, the electrode assembly identification function may be achieved with three electrical contacts by connecting the active electrode to one contact, the return electrode to another contact, and the capacitor to the third contact, with the other terminal of the capacitor connected to one of the contacts assigned to the electrodes. The capacitor may be quite small and mounted immediately distally of the contacts in a contact housing which is shaped to be received on or in a connector or handpiece which is, in turn, connected to the generator.

In the generator, the sensing means may be coupled to adjustment means in the form of a controller which is arranged to set the output power of the generator according to an output signal provided by the sensing means and representative of the identification characteristic. The controller is preferably operable to adjust the average supply voltage supplied to a radio frequency output circuit (in this case a power oscillator) in response to the sensing means output signal. In the case of the generator including a switched mode power supply, the controller is coupled to the power supply and arranged to adjust the duty cycle of the switched output of the power supply in response to the sensing means output signal.

The invention is applicable to instruments in which the generator is separate from an instrument handpiece or incorporated in the handpiece. The sensing means may be incorporated in the handpiece whether or not the generator is also in the handpiece.

According to the invention there is provided a method of assembling and operating an electrosurgical instrument comprising: providing a first unit including an electrosurgical generator for generating radio frequency power, the generator including a sensing circuit, providing a plurality of second units comprising different electrode assemblies each having means for detachable mounting to the first unit and each including an identification capacitor having a value indicative of a characteristic of the respective assembly; selecting one of the second units and mounting it to the first unit; and in the first unit, sensing the said capacitor value and automatically adjusting the generator output in response to the said sensing to suit the characteristic of the electrode assembly of the second selected unit; characterised in that the sensing circuit includes an inductance, in that the capacitor is arranged to form a resonant circuit with the inductance when the second unit is mounted to the first unit, and in that the capacitor value is sensed in the first unit by sensing the resonant frequency of the resonant circuit.

The invention includes a kit of parts for assembling an electrosurgical instrument, comprising a first unit including an electrosurgical generator for generating a radio frequency electrosurgical voltage, and a plurality of different second units including different electrode assemblies, each second unit including means for mounting to the first unit, wherein each second unit includes an identification capacitor having a respective value selected from a range of capacitance values and indicative of a characteristic of the respective electrode assembly, and the first unit includes means for sensing the indicating capacitance when the second unit is mounted to the first unit, and wherein the generator includes adjustment means responsive to the sensing means adjust the output of the generator, whereby the generator output is automatically adjusted to suit the different characteristics of the electrode assemblies of the second units when they are selectively mounted to the first unit, characterised in that the sensing means include an inductance for forming a resonant circuit with the capacitor and in that the generator output is automatically adjusted according to the resonante frequency of the resonant circuit.

The invention will now be described by way of example with reference to the drawings in which:-
Figure 1 is a diagrammatic representation of an electrosurgical instrument for urological procedures, in particular cystoscopic procedures, in which an electrode assembly and handpiece are shown partly in cross-section, and a generator is shown in block diagram form:
Figure 1A is a detail view showing the distal tip of the electrode assembly in Figure 1, partly in longitudinal cross-section:
Figure 2 is an electrical circuit diagram of an electrode assembly and an identification circuit. the latter forming part of the generator of Figure 1;
Figure 3 is an electrical circuit diagram showing the electrode assembly and an alternative identification circuit;
Figure 4 is a side elevation of an electrode assembly and connector unit of an electrosurgical instrument for hysteroscopic procedures. the electrode assembly and connector unit being shown prior to the attachment of one to the other;
Figure 5 is a partly sectioned side view of an electrode assembly and handpiece for an electrosurgical instrument for arthroscopic procedures; and
Figures 6A, 6B, and 6C are fragmentary side elevations of the electrode assemblies of the urological, hysteroscopic, and arthroscopic instruments of Figures 1, 4, and 5 respectively, showing the differences between the plug sections of these assemblies.

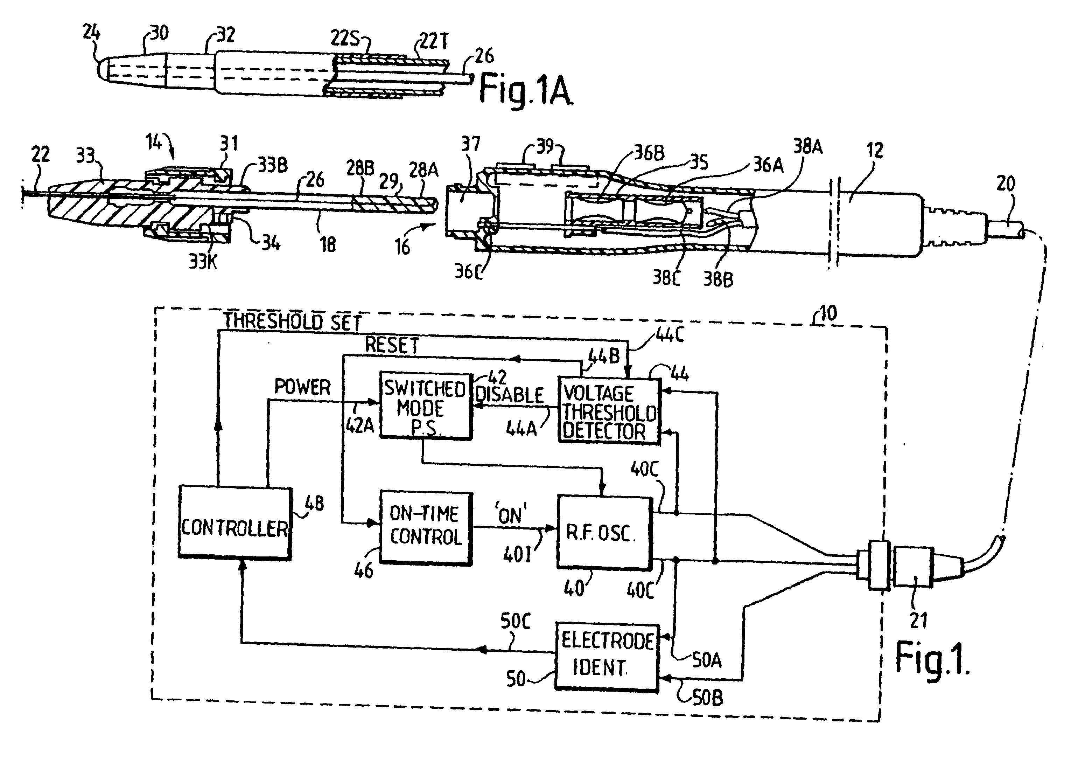

Referring to Figures 1 and 1A, an electrosurgical instrument in accordance with the invention comprises a generator 10 for generating radio frequency power, a pencil-grip handpiece 12, shown partly sectioned in Figure 1, and a detachable electrode assembly 14, shown detached from the handpiece 12 in Figure 1, but aligned with an aperture 16 of the handpiece, which receives a plug section 18 of the electrode assembly 14. In this embodiment of the invention. the generator 10 is separate from the handpiece 12, the two being connected by a cable 20 and an output connector 21, as shown.

In Figure 1, the distal end of the electrode assembly 14 is not shown. but this appears in Figure 1A to a larger scale. The assembly has a shaft 22 in the form of a conductive tube 22T covered with an insulating sheath 22S. At the extreme distal end of the shaft 22 there is an exposed central tissue contact or active electrode 24. This is a hemispherical metallic tip connected to a metallic wire which extends as a central conductor 26 through the whole of the shaft to a first contact 28A on the plug section 18 at the proximal end of the assembly 14. Surrounding the central electrode 24 is an insulating sleeve 30. the distal end of which is exposed proximally of the exposed part of the active electrode 24. Surrounding the sleeve 16 is a coaxial return electrode 32 in the form of a metallic tube which is electrically and mechanically integral with a metallic tubular body 22T of the shaft 22. The return electrode 32 is connected to a second contact 28B on the plug section 18 of the electrode assembly 14. In order that it is both radially and axially spaced from the active electrode 24, the return electrode 32 terminates at a point short of the end of the sleeve 30. In normal circumstances, only the active electrode 24 contacts the tissue to be treated; the return electrode 32 is immersed in an electrically conductive solution such as saline solution so that an electrical conducting path is formed between the tissue surrounding the active electrode 24 and the return electrode 32.

The electrode assembly plug section 18 is secured in a plastics housing 33 which also supports the shaft 22. This housing has a boss 33B coaxially surrounding the plug section 18. and a laterally projecting key portion 33K. A rotatable bayonet ring 31 secures the housing 33 to the handpiece 12.

The housing 33 contains a discrete passive electronic component, in the form of a small capacitor 34 one terminal of which is connected to the conductive tube 22T and the other terminal of which forms a third contact exposed on the proximal surface of the housing key portion 33K.

When the electrode assembly 14 is attached to the handpiece 12. the plug section 18 passes through aperture 16 and into an inner housing 35 which has spring electrical contacts 36A. 36B arranged to engage contacts 28A and 28B of the electrode assembly plug section 18. Associated with aperture 16 is a recess shaped to match the outer profile of boss 33B and key portion 33K of the electrode assembly housing 33, and a third electrical contact 33C, which is spring-loaded. is located in the recess 37 to engage the exposed terminal of capacitor 34.

Each of the three contacts 36A to 36C is connected in the handpiece 12 to respective conductors 38A. 38B. 38C of the cable 20. In this case, cable 20 has two further conductors (not shown) for connection to push-button switches 39 located in the handpiece body.

Several different electrode assembles may be provided. each having a plug section 18 and a housing 33 which fits the handpiece 12, and each having an identification element (capacitor 34) the value of which is unique to the respective electrode assembly so that the capacitance between contacts 28A and the exposed terminal of capacitor 34 identifies the respective electrode assembly.

The cable 20 is connected to the generator 10 by means of a generator output connector assembly 21 which is unique to the category of surgical procedures for which the generator output is intended, in this case urological procedures.

Referring now to the elements of the generator 10 as shown in Figure 1, a radio frequency (RF) power oscillator 40 has a pair of output connections 40C coupled to conductors 38A and 38B of cable 20 via connector 21, and thence to the active and return electrodes 24, 32 respectively of the electrode assembly 14. Power is suppled to the oscillator by a switched mode power supply 42 coupled to the oscillator 40. In the preferred embodiment, the oscillator 40 operates at about 400 kHz, with any frequency from 300 kHz upwards into the HF range being feasible. The switched mode power supply typically operates at a frequency in the range of from 25 to 50 kHz. Coupled across the output connections 40C is a voltage threshold detector 44 having a first output 44A coupled to the switched mode power supply 42 and a second output 44B coupled to an on-time control portion 46 of a control circuit. Another, controller part 48 of the control circuit, preferably configured in the form of a microprocessor controller coupled to operator controls and a display (not shown), is connected to a control input 42A of the power supply 42 and to a threshold-set input 44C of the voltage threshold detector 44.

The "on" time control circuit 46 is coupled to the RF oscillator 40 to control the period of conduction of the oscillating output device of the oscillator 40 in each cycle of radio frequency oscillation, thereby to control the power delivered to the electrode assembly 14.

The generator 10 also includes an electrode identification circuit 50 having input terminals 50A and 50B connected respectively to contacts 36B and 36C of the handpiece 12 so that the capacitor 34 in the electrode assembly, when mounted in the handpiece 12, is connected across the inputs to the electrode identification circuit 50. This circuit 50 has an output 50C coupled to an input of the controller 48.

In operation, the controller 48 causes power to be applied to oscillator 50 by the switched mode power supply 42 when electrosurgical power is demanded by the surgeon operating one of the activation switches 39 on the handpiece 12. An output voltage threshold is set via input 44C according to control settings on the front panel (not shown) of the generator 10. Typically, for desiccation, the threshold is set at a desiccation threshold value between 150 volts and 200 volts. When a cutting or vaporising output is required, the threshold is set to a value in the range of from 250 volts to 600 volts, the value being dependent on the value of the capacitor 34 in the electrode assembly 14, as represented by the output signal produced by the electrode identification circuit 50 on output 50C. The voltage values given above are peak values. The fact that they are peak values means that for desiccation at least, it is preferable to have an output radio frequency waveform of low crest factor to give maximum power before the voltage is clamped at the values given. Typically a crest factor of 1.5 or less is achieved.

When the generator is first activated, the status of the control input 40I of the oscillator 40, which is connected to the "on" time control part 46 of the control circuitry, is "on", such that the power switching device which forms the oscillating element of the oscillator 40 is switched on for a maximum conduction period during each oscillation cycle. The power delivered to the electrode assembly 14 depends partly on the supply voltage applied to the RF oscillator 40 from the switched mode power supply 42 and partly on the load impedance. The switched mode power supply 42 produces a supply voltage which is dependent on the "power" signal applied at its input 42A by the controller 48 which, in turn, depends on the front panel settings and the value of the capacitor 34 in the selected electrode assembly 14.

If the supply voltage applied to the oscillator 40 by the switched mode power supply 42 is sufficiently high, the temperature of the liquid medium surrounding the electrodes 24 and 32 may rise to an extent such that it vaporises, leading to a rapid increase in load impedance and a consequent rapid increase in the applied output voltage across the terminals 40C of the oscillator 40. This is an undesirable state of affairs if a desiccation output is required. For this reason, the threshold voltage for a desiccation output is set to cause trigger signals to be sent to the "on" time control circuit 46 and to the switched mode power supply 42 when the threshold is reached. The "on" time control circuit 46 has the effect of virtually instantaneously reducing the "on" time of the RF oscillator switching device, and simultaneously the switched mode power supply is disabled via output 44A of the detector 44 so that the voltage supplied to the oscillator 40 begins to fall.

Subsequently, the "on" time of the individual cycles of the oscillator 40 is progressively increased until the output voltage threshold is once again breached, causing a further instantaneous reduction in "on" time. As the supply voltage is reducing, the period during which the oscillator "on" time is reduced can be shortened for a given delivered output power so that, if necessary, further instantaneous power reductions can be obtained in the same way as described above. The manner in which this process is achieved is described in the above-mentioned British Patent Application No. 9512888.0.

The operation of the control circuit 46, 48 so as dynamically to control the output voltage sufficiently rapidly and to a sufficient degree to maintain the delivered power at a level suitable for desiccation can also be used in tissue cutting or vaporisation mode with a different threshold voltage dynamically to limit the output voltage to prevent electrode burning and/or excessive tissue vaporisation. In this latter case, the voltage limit may be set to a level between 250 volts and 600 volts depending on the value of capacitor 34 (Figure 1)

Both the initial power level of the RF oscillator 40 and the threshold voltage in the vaporisation mode can be adjusted according to the value of the capacitor 34 in the electrode assembly 14 using the electrode identification circuit 50 and adjustment means in the controller 48. Thus, voltage overshoot and consequent unwanted vaporisation in the desiccation mode can more easily be avoided. This is of particular concern when sealing blood vessels by desiccation. prior to cutting or vaporisation. Similarly, in the vaporisation mode, a nominal power level can automatically be set according to the electrode assembly so as to deliver a minimum power level necessary to achieve vaporisation. Also in the vaporisation mode, the maximum voltage level can be set, thereby determining the size of the vapour pocket created by the particular electrode assembly connected to the handpiece. The size of the vapour pocket in turn determines the amount of tissue removed adjacent the electrodes. Higher operating voltages, however, cause higher active electrode temperatures. Thus, if the active electrode is made of a noble metal, it is capable of withstanding a higher voltage than one constructed of less robust materials. In such circumstances, the electrode may be excessively eroded or melted, and the capacitor 34 can be used to set the voltage threshold detector in the vaporisation mode to prevent this.

With regard to adjusting the oscillator power level, the capacitor 34 is used to communicate the identity of the electrode assembly, and thus an appropriate power level, to the generator for the respective electrode assembly 14. The generator may typically have a maximum power level of 200 watts, and the minimum power level required for vaporisation for the assembly may be as low as 30 watts. To achieve an approximately logarithmic division of power levels to suit different electrode assemblies, different capacitor values can be used to represent power levels such as 30, 45, 70, 100, and 150 watts. An alternative logarithmic division is 30, 42, 58, 80, 110, and 150 watts. Capacitor values between 15pF and 1 µF can be used to indicate these power levels. If these values correspond to power thresholds, then for the desiccation mode the controller is arranged to set powers slightly below the respective values, while for vaporisation, the set values are slightly higher.

The voltage maximum for vaporisation may also be communicated, if necessary, by using further capacitor values or by including a second identification element in the electrode assembly and a fourth set of contacts and an additional conductor in the cable 20.

It is preferred that the greater the nominal power rating of the electrode assembly, the greater is the value of the capacitor 34. This allows for the possibility of failure of the electrode identification parts of the instrument, with the generator defaulting to the least power and voltage setting for safety reasons.

One benefit of setting a nominal power level according to the electrode assembly attached to the handpiece is that power can be supplied to the assembly immediately at the nominal level rather than being increased progressively to that level when the electrodes are first applied to the tissue being treated. Looked at in a different way, the surgeon can apply the required power level from the start, with much reduced danger of electrode damage or unwanted tissue disruption.

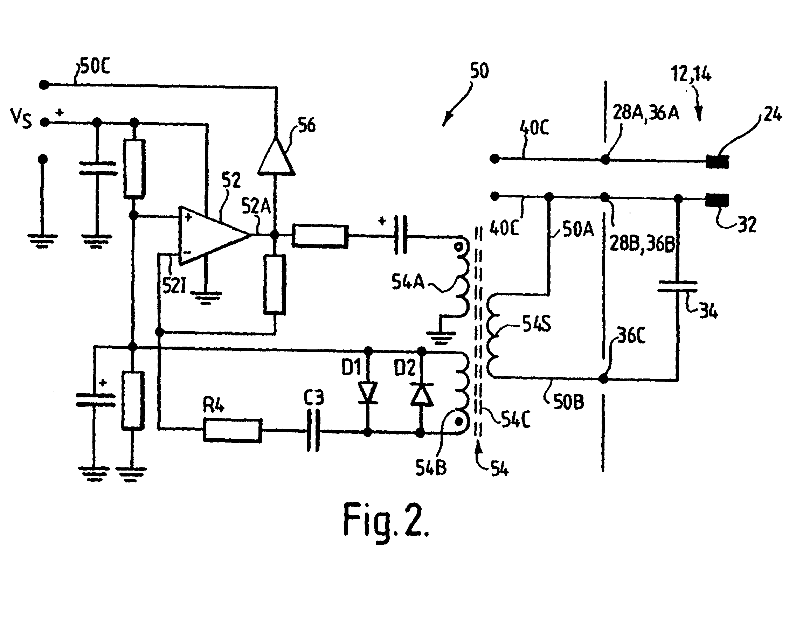

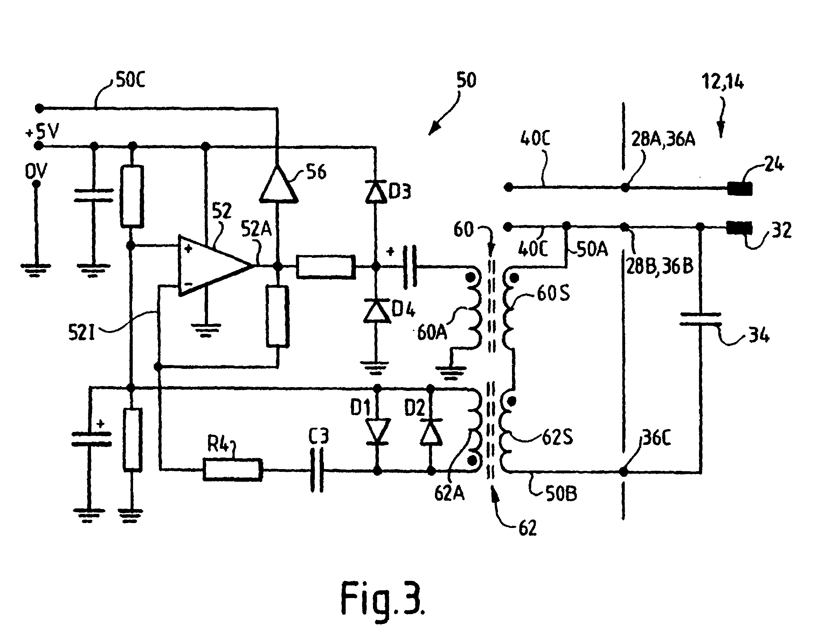

The electrode identification circuit will now be described in more detail with reference to Figures 2 and 3.

The electrode identification circuit 50 is centred on an operational amplifier 52 having a low impedance output 52A driving an excitation primary winding 54A of an isolation transformer 54. A secondary winding 54S of the transformer 54 is coupled across the input leads 50A and 50B of the circuit 50 so that winding 54S and capacitor 34 of the electrode assembly form a parallel resonant circuit. The resonant frequency of the resonant circuit is typically within the range of from 2 kHz to 150 kHz, depending on the value of capacitor 34.

The transformer 54 also has a sense winding 54B coupled between an AC ground on one side and the inverting input 521 of the operational amplifier 52, thereby providing a feedback path from the transformer. Since winding 54B is effectively coupled to the excitation winding 54A via the resonant secondary winding 54S, the presence of the resonant circuit largely filters out the harmonics of the square wave output of the operational amplifier 52.

Clamp diodes D1 and D2 connected with opposite pluralities across sense winding 54B provide, in conjunction with capacitor C3 and resistor R4, a phase shift network causing a 90 degree phase lag with respect to the excitation winding output. The diodes also provide protection against excessive radio frequency voltages received from the generator radio frequency output applied via conductors 38A and 38B.

The three windings 54A, 54B and 54S of transformer 54 are wound on a three-section bobbin with a central threaded iron dust core 54C, this material being chosen due to its high curie point and consequent minimal thermal drift. Alternatively, core 54C may be made of a ferrite material with a comparatively large Al value in conjunction with a calibration reference to allow compensation for thermal drift by, for example, switching in a known capacitance across the resonant winding 54S.

Coupling between the resonant secondary winding 54S and the other windings 54A, 54B of the transformer 54 is comparatively low to limit radio frequency feedback. Typically, the leakage inductance is in the region of 3 mH.

It will be appreciated from the above that operational amplifier 52 acts as an oscillator, oscillating at the resonant frequency of the resonant circuit produced by secondary winding 54S and capacitor 34. The output signal produced by the operational amplifier 52 is amplified in a buffer amplifier 56 and applied to output terminal 50C from where it is fed to the controller 48 (see Figure 1). Controller 48 contains a counter for determining the frequency of oscillation or an equivalent measurement from which the identity of the electrode assembly is obtained.

As a safety feature the controller 48 includes means for determining from the output of the identification circuit 50 whether any electrode assembly is connected to the handpiece 12. In such an eventuality, the oscillation frequency of the circuit 50 is outside a predetermined range (in this embodiment it is higher than 150 kHz) and the adjusting means generates a signal indicative of no electrode assembly being connected and the supply of RF output power to the handpiece 12 is inhibited.

In an alternative embodiment, shown in Figure 3, the electrode identification circuit 50 has two isolation transformers 60 and 62 to avoid magnetic coupling between an excitation winding 60A coupled to the operational amplifier output 52A on the one hand, and a sense winding 62A coupled to the non-inverting input 52I of the operational amplifier on the other hand. Secondary windings 60S and 62S of the two transformers are coupled in series, their combined inductance forming a parallel resonant circuit with electrode assembly capacitor 34. Compared with the circuit of Figure 2, the lack of magnetic coupling as a result of the dual transformer arrangement reduces the transmission of harmonics to the feedback loop of the oscillator. Thus, all energy supplied into excitation winding 60A is filtered in such a way that only filtered energy arrives at the sense winding 62A.

In this embodiment, two additional diodes D3 and D4 are used to clamp and protect the operational amplifier 52 from inadvertent radio frequency inputs on the third conductor 50B (e.g. by misuse of the electrode assembly and handpiece or due to insulation failure).

In other respects, the identification circuit of Figure 3 corresponds to that of Figure 2.

In the above detailed description we have used the example of an electrosurgical instrument intended for urological procedures, in particular cystoscopic procedures. The invention is equally applicable to electrosurgical instruments in other fields, such as hysteroscopic and arthroscopic procedures.

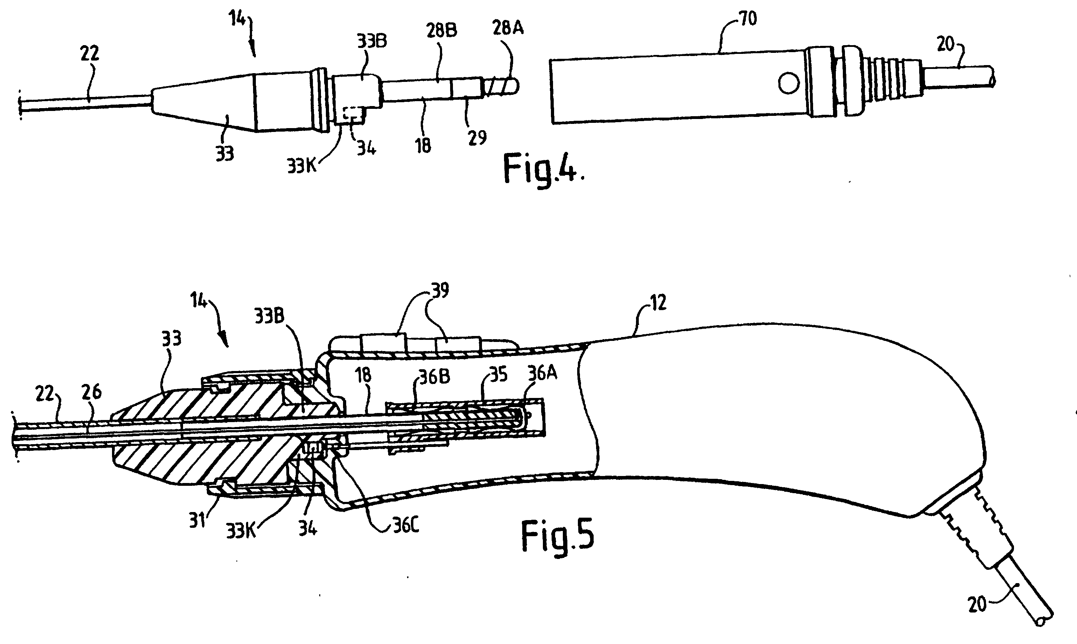

Parts of an instrument for hysteroscopic use are shown in Figure 4. In this case, an electrode assembly 14 (which may have an electrode configuration similar to that shown in Figure 1A) has a plug section 18 and housing 33 very similar to the corresponding parts of the urological instrument of Figure 1. As in the urological instrument, a capacitor 34 (here shown by dotted lines) is positioned in a laterally projecting key portion 33K of the housing 33 to contact a spring-loaded contact similar to contact 36C of Figure 1 in a connector unit 70 attached to cable 20.

Connector unit 70 is typically attached to the outside of an endoscope (not shown).

Referring to Figure 5, an arthroscopic instrument has a handpiece 12 ergonomically designed to aid tissue manipulation. With regard to the interengagement of the handpiece 12 and electrode assembly 14, these parts have features similar to the features described with reference to the urological instrument of Figure 1.

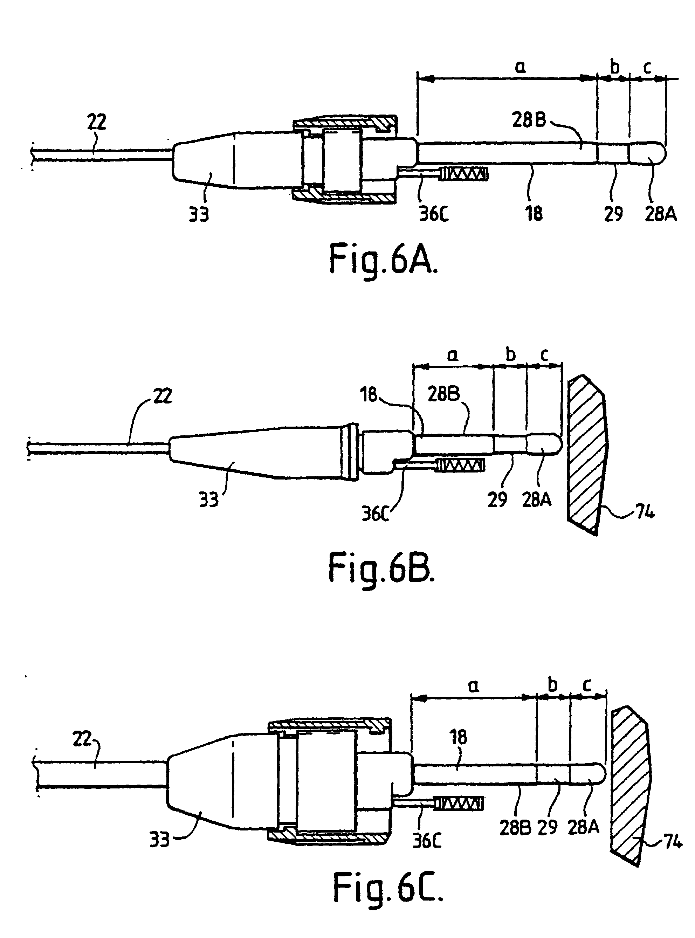

It will be seen by comparing the electrode assemblies 14 and handpieces 12 and connector unit 70 of the instruments shown in Figures 1, 4, and 5, that the respective plug sections 18 are of different lengths. In each case, the receptacle 35 has contacts 36A and 36B for engaging contacts 28A and 28B of the plug section 18 which are located according to the length of the corresponding electrode assembly plug section 18. These differences are shown more clearly in Figures 6A, 6B, and 6C which show the urological, hysteroscopic, and arthroscopic electrode assemblies respectively. In all three figures, the spring loaded contact 36C for engaging the terminal of the capacitor 34 is also shown. Furthermore, in Figures 6B and 6C a stop 74 is shown diagrammatically. This is for limiting insertion of plug section 18 and, in practice, is typically provided in those instruments by means of a transversely oriented dowel in the receptacle 35.

It will be noted that although the lengths *c* and *b* of the contact 28A at the end of the plug unit 18, and the insulative spacer 29 remain the same in all three embodiments, the distance a between the housing 33 and the insulative spacer 29 is different in each case. The same dimensions are maintained in all electrode assemblies within each speciality group. Thus, all electrode assemblies intended for urological procedures have a relatively long plug section 18, all electrode assemblies for hysteroscopic procedures have a short plug section 18, while all those intended for arthroscopic procedures have a medium length plug section 18, as shown in Figures 6A, 6B, and 6C. Consequently, each electrode assembly can only be used with a handpiece 12 or connector unit 70 intended for the same speciality group. If there is a mismatch in the mechanical interface between an electrode assembly and a handpiece or connector unit, electrosurgical power cannot be supplied from the generator to the electrodes either because one or both of the contacts 28A, 28B fails to make contact with a corresponding contact in the handpiece or connector unit, or because the spring-loaded contact 36C does not engage with the identification capacitor 34 in the housing 33.

By providing similar interface restrictions at the connector 21 (see Figure 1), it can be ensured that each electrode assembly can only be used with a generator output connection configured for the speciality group of the electrode assembly. As a result, it is possible to set up the generator differently according to speciality grouping, and so that it responds differently to the various identification capacitor values within the range of electrode assemblies for that speciality grouping. This means that a wider range of generator settings is available than would be possible by relying entirely on a limited range of values for capacitor 34.

## Claims

1. An electrosurgical instrument comprising a first unit including a generator (10) for generating radio frequency power, and a second unit including an electrode assembly (14), the second unit being detachably connectible to the first unit such that radio frequency power can be conveyed to the electrode assembly (14), the second unit including an identification capacitor (34) having a value indicative of a characteristic of the electrode assembly (14), and the first unit includes sensing means (50) for sensing the value of the capacitor (34) when the second unit is connected to the first unit, the generator (10) including adjustment means (48) responsive to the sensing means to adjust the output of the generator so as to suit the indicated electrode assembly characteristic, and **characterised in that** the sensing means (50) includes an inductance (54S, 60S, 62S) which forms a resonant circuit with the identification capacitor (34) when the second unit is connected to the first unit, the resonant frequency of the resonant circuit being dependent on the value of the capacitor (34).

2. An instrument according to claim 1, **characterised in that** the sensing means (50) and the identification capacitor (34) together form an oscillator of which the frequency of operation is dependent on the value of the identification capacitor (34).

3. An instrument according to claim 2, **characterised in that** the inductance (54S) comprises a first winding (54S, 62S) of a transformer (54, 62), the identification capacitor (34) is connected between a first pair of contacts on the second unit, and the transformer first winding (54S, 62S) is connected between a second pair of contacts (36B, 36C) on the first unit, the first and second pairs of contacts being so located that the contacts of one pair engage respective contacts of the other pair when the first unit is connected to the second unit to form the said resonant circuit.

4. An instrument according to claim 3, **characterised in that** the transformer (54, 62) is an isolation transformer and has a second winding (54B, 62A) forming part of the oscillator.

5. An instrument according to claim 4, **characterised in that** the transformer (54) has a third winding (54A) coupled to an oscillating device (52) of the oscillator and acting as a resonant circuit excitation winding.

6. An instrument according to claim 4, **characterised by** a second transformer (60) having a first winding (60A) coupled to an oscillating device (52) of the oscillator and acting as a resonate circuit containing winding, and a second winding (60S) coupled in series in the resonant circuit exicitation winding, and a second winding (60S) coupled in series in the resonant circuit.

7. An instrument according to any of claims 3 to 6, **characterised in that** one of the respective contacts (28B, 36B) of each of the first and second pairs also serves as a contact for conducting radio frequency electrosurgery currents between the generator (10) and the electrode assembly (14).

8. An instrument according to any of claims 2 to 7, **characterised in that** the sensing means (50) includes a phase shift network (D1, D2, C3, R4) in a feedback path of the oscillator.

9. An instrument according to claim 8, **characterised in that** the phase shift network (D1, D2, C3, R4) provides a 90° phase shift.

10. An instrument according to claim 9, **characterised in that** the phase shift network comprises a pair of clamp diodes (D1, D2) connected with opposite polarities, and the series combination of a capacitor (C3) and a resistor (R4).

11. An instrument according to any preceding claim, **characterised in that** the sensing means (50) are responsive to a plurality of different values of the identification capacitor (34), and **in that** the adjustment means comprise a controller (48) arranged to set the output power of the generator according to an output signal provided by the sensing means (50) and representative of the identification capacitor value.

12. An instrument according to claim 11, **characterised in that** the controller (48) is operable to adjust the average supply voltage supplied to a radio frequency output circuit of the generator in response to the output signal of the sensing means (50).

13. An instrument according to claim 12, **characterised in that** the generator (10) includes a switched mode power supply (42), and the controller (48) is coupled to the power supply (42) and operable to adjust the duty cycle of the switched output in response to the output signal of the sensing means (50).

14. An instrument according to any preceding claim, **characterised in that** the first unit comprises the generator (10), a connector (36A, 36B, 36C), and a cable (20) for coupling the generator (10) to the connector (36A, 36B, 36C), the cable (20) including conductors for coupling the identification capacitor (34) to the sensing means (50), and **in that** the second unit is in the form of an electrode assembly (14) including a connector which mates with the connector (36A, 36B, 36C) of the first unit.

15. An instrument according to claim 14, **characterised in that** the said connector (36A, 36B, 36C) of the first unit is integrated in a handpiece (12) of the instrument.

16. An instrument according to any preceding claim, **characterised in that** the adjustment means (48) includes a circuit for setting a generator output voltage limit for tissue vaporisation, the limit being determined according to the identification component property.

17. An instrument according to any preceding claim, **characterised in that** the sensing means includes a calibration reference capacitance and a switch for switching the reference capacitance across the inductance.

18. An instrument according to claim 1, **characterised in that** the sensing means (50) includes an oscillator for exciting a resonance in the combination of the identification capacitor and the inductance when the second unit is connected to the first unit, the oscillator having a transformer-coupled output for providing d.c. isolation of the capacitor.

19. An instrument according to claim 18, **characterised in that** the identification capacitor (24) is coupled to the transformer-coupled output of the oscillator by first and second pairs of contacts, one pair of contacts forming part of the first unit and the other forming part of the second unit, and **in that** one (28B, 36B) of each of the two pairs of contacts also serves for the conduction of radio frequency electrosurgical currents between the generator (10) and the electrode assembly (14), and the other contact of each pair of contacts is an identification contact.

20. An instrument according to claim 19, **characterised in that** each of the said first and second unit has only a single said identification contact.

21. An electrosurgical generator for use with a plurality of different electrode assemblies including respective identification capacitors having different values indicative of the characteristics of the electrode assemblies, the generator (10) including sensing means (50) responsive to the capacitor values and means (48) for automatically setting the output of the generator according to the indicated characteristic of a selected electrode assembly connected to the generator, **characterised in that** the sensing means (50) includes an inductance (54S, 60S, 62S) arranged to form a resonant circuit with the capacitor of each selected electrode assembly, the resonant frequency of which resonant circuit is dependent on the respective capacitor value, and the sensing means (50) are operable to produce an electrical output signal for feeding to the setting means (48), the nature of which output signal is dependent on the resonant frequency and, therefore, on the value of the capacitor of the selected electrode assembly.

22. An generator according to claim 21, **characterised in that** the automatic setting means comprises a controller (48) arranged to set at least one of the output power of the generator and a maximum peak output voltage.

23. A generator according to claim 21 or claim 22, **characterised in that** the inductance (54S, 60S, 62S) forms part of an oscillator arranged such that its oscillation frequency is the said resonant frequency.

24. A generator according to claim 23, **characterised in that** the oscillator has a transformer-coupled output to provide d.c. isolation of the identification capacitor of a selected electrode assembly when connected to the generator.

25. A generator according to claim 24, **characterised in that** the inductance comprises a winding (54S, 62S) of an isolation transformer (54, 62) providing the transformer-coupled output of the oscillator.

26. A generator according to any of claims 21 to 25, **characterised by** a plurality of contacts (36A, 36B, 36C) for mating with a respective plurality of contacts associated with a selected electrode assembly, and **characterised in that** the inductance (54S, 60S, 62S) is coupled across first and second contacts (36B, 36C) of the said plurality of contacts of the generator, the first contact constituting a single identification contact (36C) and the second (36B) being so coupled as to form part of the current path for delivering electrosurgical currents to the electrode assembly.

27. A generator according to claim 21, **characterised in that** the sensing means (50) includes an oscillator for exciting a resonance in the identification capacitor of the selected assembly and the inductance (54S, 60S, 62S) forming part of the combination of the generator and the electrode assembly, the oscillator having a transformer-coupled output for providing d.c. isolation of the identification capacitor.

28. A generator according to claim 27, **characterised by** a pair of contacts (36B, 36C) for mating with a respective pair of contacts of each selected electrode assembly, the transformer-coupled output of the oscillator being coupled to the said pair of contacts of the generator, wherein one (36B) of the pair of generator contacts also serves for the conduction of radio electrosurgical currents to the electrode assembly, the other contact (36C) constituting an identification contact

29. A generator according to claim 28, **characterised by** only a single said identification contact (36C).

30. A method of assembling and operating an electrosurgical instrument comprising:
providing a first unit including an electrosurgical generator (10) for generating radio frequency power, the generator including a sensing circuit (50),
providing a plurality of second units comprising different electrode assemblies (14) each having means for detachable mounting to the first unit and each including an identification capacitor (34) having a value indicative of a characteristic of the respective assembly;
selecting one of the second units and mounting it to the first unit; and
in the first unit, sensing the said capacitor value and automatically adjusting the generator output in response to the said sensing to suit the characteristic of the electrode assembly of the second selected unit;
**characterised in that** the sensing circuit (50) includes an inductance (54S; 60S, 65S), **in that** the capacitor (34) is arranged to form a resonant circuit with the inductance when the second unit is mounted to the first unit, and **in that** the capacitor value is sensed in the first unit by sensing the resonant frequency of the resonant circuit.

31. A method according to claim 30, **characterised in that** the generator nominal output power is automatically adjusted according to the sensed identification parameter value.

32. A method according to claim 30, or claim 31, **characterised in that** a tissue vaporisation limit voltage is automatically adjusted according to the sensed identification parameter value.

33. A method according to claim 30, **characterised in that** a characteristic of the generator output is adjusted in response to the identification component parameter value according to the category of surgical procedure as **characterised by** the mechanical configuration of the generator output connector to which the electrode assembly is connected.

34. A kit of parts for assembling an electrosurgical instrument, comprising a first unit including an electrosurgical generator (10) for generating a radio frequency electrosurgical voltage, and a plurality of different second units including different electrode assemblies (14), each second unit including means (31, 33) for mounting to the first unit, wherein each second unit includes an identification capacitor (34) having a respective value selected from a range of capacitance values and indicative of a characteristic of the respective electrode assembly (14), and the first unit includes means (50) for sensing the indicating capacitance when the second unit is mounted to the first unit, and wherein the generator (10) includes adjustment means responsive to the sensing means adjust the output of the generator, whereby the generator output is automatically adjusted to suit the different characteristics of the electrode assemblies of the second units when they are selectively mounted to the first unit, **characterised in that** the sensing means include an inductance (54S, 60S, 62S) for forming a resonant circuit with the capacitor (34) and **in that** the generator output is automatically adjusted according to the resonant frequency of the resonant circuit.

35. A kit of parts according to claim 34, **characterised in that** the first unit comprises the generator (10), a connector (36A, 36B, 36C), and a cable (20) for coupling the generator to the cable, and wherein each second unit comprises a said electrode assembly (14) which itself includes a connector for mating with the connector (36A, 36B, 36C) of the first unit.

## Patentansprüche

1. Elektrochirurgisches Gerät mit einer einen Generator (10) umfassenden ersten Einheit zur Erzeugung von Hochfrequenz-Sendeleistung und einer zweiten Einheit mit einer Elektrodenvorrichtung (14), wobei die zweite Einheit mit der ersten Einheit derart lösbar verbindbar ist, dass die Sendeleistung an die Elektrodenvorrichtung (14) übertragbar ist und die zweite Einheit einen Kennkondensator (34) mit einem auf einen Kennwert der Elektrodenvorrichtung (14) hinweisenden Wert umfasst, die erste Einheit Erfassungsmittel (50) zur Ermittlung des Werts des Kondensators (34) bei Verbindung der zweiten Einheit mit der ersten Einheit umfasst und der Generator (10) auf die Erfassungsmittel ansprechende Einstellmittel (48) zur Abstimmung der Generatorleistung umfasst, so dass diese zu dem angezeigten Kennwert der Elektrodenvorrichtung passt, **dadurch gekennzeichnet, dass** das Erfassungsmittel (50) eine Induktivität (54S, 60S, 62S) aufweist, die, wenn die zweite Einheit mit der ersten verbunden ist, zusammen mit dem Kennkondensator (34) einen Resonanzkreis bildet, wobei die Resonanzfrequenz des Resonanzkreises von dem Wert des Kondensators (34) abhängig ist.

2. Gerät nach Anspruch 1, **dadurch gekennzeichnet, dass** das Erfassungsmittel (50) und der Kennkondensator (34) zusammen einen Oszillator bilden, dessen Arbeitsfrequenz von dem Wert des Kennkondensators (34) abhängig ist.

3. Gerät nach Anspruch 2, **dadurch gekennzeichnet, dass** die Induktivität (54S) eine Primärwicklung (54S, 62S) eines Transformators (54, 62) aufweist, der Kennkondensator (34) zwischen einem ersten Paar von Kontakten an der zweiten Einheit angeschlossen ist und die Primärwicklung (54S, 62S) des Transformators zwischen einem zweiten Paar von Kontakten (36B, 36C) an der ersten Einheit angeschlossen ist, wobei die ersten und zweiten Paare von Kontakten derart angeordnet sind, dass, wenn die erste Einheit mit der zweiten Einheit zur Bildung des Resonanzkreises verbunden wird, die Kontakte des einen Paars mit den entsprechenden Kontakten des anderen Paars ineinander greifen.

4. Gerät nach Anspruch 3, **dadurch gekennzeichnet, dass** der Transformator (54, 62) ein Isolationstransformator ist und eine Sekundärwicklung (54B, 62A) aufweist, die einen Teil des Oszillators bildet.

5. Gerät nach Anspruch 4, **dadurch gekennzeichnet, dass** der Transformator (54) eine dritte Wicklung (54A) aufweist, die mit einer Schwingeinrichtung (52) des Oszillators gekoppelt ist und als Anregungswicklung des Resonanzkreises fungiert.

6. Gerät nach Anspruch 4, **gekennzeichnet durch** einen zweiten Transformator (60) mit einer an eine Schwingeinrichtung (52) des Oszillators gekoppelten Primärwicklung (60A), die als eine einen Resonanzkreis beinhaltende Wicklung fungiert und mit einer Sekundärwicklung (60S), die in der Anregungswicklung des Resonanzkreises in Reihe geschaltet ist sowie einer Sekundärwicklung (60S), die in dem Resonanzkreis in Reihe geschaltet ist.

7. Gerät nach einem der Ansprüche 3 bis 6, **dadurch gekennzeichnet, dass** einer der jeweiligen Kontakte (28B, 36B) jedes der ersten und zweiten Paare auch als Kontakt zur Leitung von elektrochirurgischen Hochfrequenzströmen zwischen dem Generator (10) und der Elektrodenvorrichtung (14) dient.

8. Gerät nach einem der Ansprüche 2 bis 7, **dadurch gekennzeichnet, dass** das Erfassungsmittel (50) eine Phasenschieberanordnung (D1, D2, C3, R4) im Rückkoppelpfad des Oszillators aufweist.

9. Gerät nach Anspruch 8, **dadurch gekennzeichnet, dass** die Phasenschieberanordnung (D1, D2, C3, R4) eine Phasenverschiebung von 90° liefert.

10. Gerät nach Anspruch 9, **dadurch gekennzeichnet, dass** die Phasenschieberanordnung ein Paar von mit entgegengesetzten Polaritäten verbundenen Klemmdioden (D1, D2) und eine Reihenschaltung eines Kondensators (C3) und eines Widerstands (R4) aufweist.

11. Gerät nach einem der vorhergehenden Ansprüche, dadurch**gekennzeichnet**, dass das Erfassungsmittel (50) auf eine Mehrzahl verschiedener Werte des Kennkondensators anspricht und dass das Einstellmittel einen Regler (48) aufweist, der dafür eingerichtet ist, die Ausgangsleistung des Generators entsprechend einem durch das Erfassungsmittel (50) gelieferten und für den Wert des Kennkondensators typischen Ausgangssignal einzustellen.

12. Gerät nach Anspruch 11, **dadurch gekennzeichnet, dass** der Regler (48) zur Anpassung der Versorgungsspannung des Ausgangskreises des Generators als Ansprechen auf ein Ausgangssignal des Erfassungsmittels (50) betreibbar ist.

13. Gerät nach Anspruch 12, **dadurch gekennzeichnet, dass** der Generator (10) eine schaltbare Spannungsversorgung (42) umfasst und der Regler (48) an die Spannungsversorgung (42) gekoppelt und zur Anpassung der Schaltdauer der geschalteten Ausgangsleistung als Ansprechen auf ein Ausgangssignal des Erfassungsmittels (50) betreibbar ist.

14. Gerät nach einem der vorhergehenden Ansprüche, **dadurch gekennzeichnet, dass** die erste Einheit den Generator (10), ein Verbindungsstück (36A, 36B, 36C) und ein Kabel (20) zur Kopplung des Generators (10) an das Verbindungsstück (36A, 36B, 36C) aufweist, wobei das Kabel (20) Leiter zur Kopplung des Kennkondensators (34) an das Erfassungsmittel (50) aufnimmt und dass die zweite Einheit in Form einer Elektrodenvorrichtung (14) vorliegt, die ein Verbindungsstück umfasst, welches an das Verbindungsstück (36A, 36B, 36C) der ersten Einheit angepasst ist.

15. Gerät nach Anspruch 14, **dadurch gekennzeichnet, dass** das besagte Verbindungsstück (36A, 36B, 36C) der ersten Einheit in ein Handteil (12) des Geräts integriert ist.

16. Gerät nach einem der vorhergehenden Ansprüche, dadurch**gekennzeichnet**, dass das Einstellmittel (48) einen Schaltkreis zur Einstellung eines Grenzwerts der Generatorausgangsspannung für die Verdampfung von Gewebe aufweist, wobei der Grenzwert entsprechend der Eigenschaft der Kennkomponente ermittelbar ist.

17. Gerät nach eine der vorhergehenden Ansprüche, **dadurch gekennzeichnet, dass** das Erfassungsmittel eine Referenzkapazität zur Kalibrierung und einen Schalter aufweist, mittels dessen die Referenzkapazität auf die Induktivität schaltbar ist.

18. Gerät nach Anspruch 1, **dadurch gekennzeichnet,dass** das Erfassungsmittel (50) einen Oszillator zur Anregung einer Resonanz in der Verbindung des Kennkondensators und der Induktivität bei Verbindung der zweiten Einheit mit der ersten Einheit umfasst, wobei der Oszillator einen Transformator-gekoppelten Ausgang zur Gleichstromisolation des Kondensators aufweist.

19. Gerät nach Anspruch 18, **dadurch gekennzeichnet,dass** der Kennkondensator (24) durch erste und zweite Paare von Kontakten an den Transformator-gekoppelten Ausgang des Oszillators gekoppelt ist, wobei das eine Paar von Kontakten einen Teil der ersten Einheit, das andere einen Teil der zweiten Einheit bilden und dass eines (28B, 36B) von jedem der beiden Paare von Kontakten auch der Leitung von elektrochirurgischen Strömen zwischen dem Generator (10) und der Elektrodenvorrichtung (14) dient und der andere Kontakt jedes Paars von Kontakten ein Kennkontakt ist.

20. Gerät nach Anspruch 19, **dadurch gekennzeichnet,dass** von der ersten und zweiten Einheit jede jeweils nur einen von besagten Kennkontakten aufweist.

21. Elektrochirurgischer Generator zur Verwendung mit einer Mehrzahl von Elektrodenvorrichtungen, die jeweils verschiedene Kennkondensatoren mit unterschiedlichen Werten umfassen, welche die unterschiedlichen Kennwerte der Elektrodenvorrichtungen anzeigen, wobei der Generator (10) auf die Kondensatorwerte ansprechende Erfassungsmittel (50) und Mittel (48) zur automatischen Einstellung der Ausgangsleistung des Generators entsprechend des angezeigten Kennwerts einer ausgewählten, mit dem Generator verbundenen Elektrodenvorrichtung aufweist, **dadurch gekennzeichnet, dass** das Erfassungsmittel (50) eine Induktivität (54S, 60S, 62S) aufweist, die mit dem Kondensator jeder Elektrodenvorrichtung einen Resonanzkreis bildet, dessen Resonanzfrequenz von dem jeweiligen Kondensatorwert abhängt und die Erfassungsmittel (50) ein elektrisches Ausgangssignals zur Speisung der Einstellmittel (48) erzeugen können, wobei die Art des Ausgangssignals von der Resonanzfrequenz und demgemäss von dem Wert des Kondensators der ausgewählten Elektrodenvorrichtung abhängt.

22. Generator nach Anspruch 21, **dadurch gekennzeichnet,dass** das automatische Einstellmittel einen Regler (48) aufweist, der dazu vorgesehen ist, zumindest entweder die Ausgangsleistung des Generators oder die Ausgangs-Peakspannung einzustellen.

23. Generator nach Anspruch 21 oder 22, **dadurch gekennzeichnet,dass** die Induktivität (54S, 60S, 62S) einen Teil eines Oszillators bildet, der derart vorgesehen ist, dass dessen Oszillationsfrequenz die besagte Resonanzfrequenz ist.

24. Generator nach Anspruch 23, **dadurch gekennzeichnet,dass** der Oszillator einen Transformator-gekoppelten Ausgang zur Gleichstromisolation des Kennkondensators einer ausgewählten Elektrodenvorrichtung bei Verbindung mit dem Generator aufweist.

25. Generator nach Anspruch 24, **dadurch gekennzeichnet,dass** die Induktivität eine Wicklung (54S, 62S) eines Isolationstransformators (54, 62) aufweist, der den Transformator-gekoppelten Ausgang des Oszillators bereitstellt.

26. Generator nach einem der Ansprüche 21 bis 25, **gekennzeichnet durch** eine Mehrzahl von Kontakten (36A, 36B, 36C),die zu einer entsprechenden Mehrzahl von einer ausgewählten Elektrodenvorrichtung zugeordneten Kontakten passen und weiter **dadurch** gekennzeichnet, dass die Induktivität (54S, 60S, 62S) über die ersten und zweiten Kontakte (36B, 36C) der Mehrzahl von Kontakten des Generators gekoppelt ist, wobei der erste Kontakt einen einzelnen Kennkontakt (36C) bildet und der zweite (36B) so gekoppelt ist, dass er einen Teil des Strompfads bildet, der elektrochirurgische Ströme an die Elektrodenvorrichtung liefert.

27. Generator nach Anspruch 21, **dadurch gekennzeichnet,dass** das Erfassungsmittel (50) einen Oszillator zur Anregung einer Resonanz in dem Kennkondensator der ausgewählten Elektrodenvorrichtung umfasst, die Induktivität (54S, 60S, 62S) einen Teil der Verbindung des Generators und der Elektrodenvorrichtung bildet und der Oszillator einen Transformator-gekoppelten Ausgang zur Gleichstromisolation des Kennkondensators aufweist.

28. Generator nach Anspruch 27, **gekennzeichnet durch** ein Paar von Kontakten (36B, 36C), die zu einem entsprechenden Paar von Kontakten jeder ausgewählten Elektrodenvorrichtung passen und **dadurch**, dass der Transformator-gekoppelte Ausgang des Oszillators mit dem Kontaktpaar des Generators gekoppelt ist, wobei einer der Generatorkontakte (36B) auch der Leitung von elektrochirurgischen Strömen zu der Elektrodenvorrichtung dient und der andere Kontakt (36C) einen Kennkontakt bildet.

29. Generator nach Anspruch 28, **dadurch gekennzeichnet, dass** er lediglich einen dieser Kennkontakte (36C) aufweist.

30. Verfahren zur Montage und zum Betrieb eines elektrochirurgischen Geräts umfassend das Bereitstellen einer ersten Einheit mit einem elektrochirurgischen Generator zur Erzeugung von Radiofrequenz-Sendeleistung, wobei der Generator einen Erfassungsschaltkreis (50) aufweist, das Bereitstellen einer Mehrzahl von zweiten Einheiten mit unterschiedlichen Elektrodenvorrichtungen (14), von denen jede Mittel zur lösbaren Anbringung an die erste Einheit aufweist und jede einen Kennkondensator (34) mit einem auf einen Kennwert der jeweiligen Vorrichtung hinweisenden Wert aufweist; Auswählen einer der zweiten Einheiten und deren Anbringung an der ersten Einheit; und die erste Einheit, mit der der Wert des Kondensators erfasst und automatisch als Reaktion auf die Erfassung die Generatorausgangsleistung abgestimmt wird, so dass diese zu dem Kennwert der Elektrodenvorrichtung der gewählten zweiten Einheit passt; **dadurch gekennzeichnet, dass** der Erfassungsschaltkreis (50) eine Induktivität (54S, 60S, 65S) aufweist, dass der Kondensator (34) so angeordnet wird, dass er bei Anbringung der zweiten Einheit an der ersten Einheit mit der Induktivität einen Resonanzkreis bildet und dass der Kondensatorwert in der ersten Einheit durch Erfassung der Resonanzfrequenz des Resonanzkreises erfasst wird.

31. Verfahren nach Anspruch 30, **dadurch gekennzeichnet, dass** die Nennausgangsleistung des Generators automatisch entsprechend des erfassten Kennparameterwerts abgestimmt wird.

32. Verfahren nach Anspruch 30 oder 31, **dadurch gekennzeichnet, dass** eine Grenzspannung zur Verdampfung von Gewebe automatisch entsprechend des erfassten Kennparameterwerts abgestimmt wird.

33. Verfahren nach Anspruch 30, **dadurch gekennzeichnet, dass** ein Kennwert der Generatorausgangsleistung als Antwort auf den Komponentenkennparameterwert entsprechend der Kategorie desjenigen chirurgischen Verfahrens abgestimmt wird, dass durch die mechanische Konfiguration des Verbindungsstücks des Generatorausgangs charakterisiert wird, an das die Elektrodenvorrichtung angeschlossen wird.

34. Satz von Teilen zur Montage eines elektrochirurgischen Geräts mit einer ersten Einheit, die einen elektrochirurgischen Generator (10) zur Erzeugung elektrochirurgischer Hochfrequenz-Spannung aufweist und einer Mehrzahl unterschiedlicher zweiter Einheiten mit verschiedenen Elektrodenvorrichtungen (14), wobei jede zweite Einheit Mittel (31, 33) zur Anbringung an die erste Einheit umfasst, jede zweite Einheit einen Kennkondensator (34) mit einem verschiedenen Kapazitätswert aufweist, der aus einem Wertebereich von Kapazitätswerten ausgewählt ist und auf einen Kennwert der jeweiligen Elektrodenvorrichtung (14) hinweist und wobei die erste Einheit Mittel (50) zur Erfassung der Kennkapazität bei. Anbringung der zweiten Einheit an der ersten Einheit aufweist und der Generator (10) auf die Erfassungsmittel ansprechende Einstellmittel zur Einstellung der Ausgangsleistung des Generators umfasst, wobei diese automatisch abstimmbar ist, so dass sie zu den unterschiedlichen Kennwerten der Elektrodenvorrichtungen der zweiten Einheiten bei deren selektiver Anbringung an der ersten Einheit passt, **dadurch gekennzeichnet, dass** die Erfassungsmittel eine Induktivität (54S, 60S, 62S) zur Bildung eines Resonanzkreises mit dem Kondensator (34) aufweisen und dass die Generatorausgangsleistung automatisch entsprechend der Resonanzfrequenz des Resonanzkreises abstimmbar ist.

35. Satz von Teilen nach Anspruch 34, **dadurch gekennzeichnet, dass** die erste Einheit einen Generator (10), ein Verbindungsstück (36A, 36B, 36C) und ein Kabel (20) zur Kopplung des Generators an das Kabel aufweist, und dass jede zweite Einheit eine Elektrodenvorrichtung (14) aufweist, die ihrerseits ein Verbindungsstück umfasst, welches zu dem Verbindungsstück (36A, 36B, 36C) der ersten Einheit passt.

## Revendications

1. Instrument électrochirurgical comprenant une première unité comportant un générateur (10) pour générer une puissance radiofréquence, et une seconde unité comportant un ensemble électrode (14), la seconde unité étant connectable de manière détachable à la première unité de telle sorte qu'une puissance radiofréquence peut être transmise à l'ensemble électrode (14), la seconde unité comportant un condensateur d'identification (34) ayant une valeur indicative d'une caractéristique de l'ensemble électrode (14), et la première unité comportant des moyens de détection (50) pour détecter la valeur du condensateur (34) lorsque la seconde unité est connectée à la première unité, le générateur (10) comportant des moyens d'ajustement (48) sensibles aux moyens de détection pour ajuster la sortie du générateur de manière à ce qu'elle s'adapte à la caractéristique indiquée de l'ensemble électrode, et **caractérisé en ce que** les moyens de détection (50) comportent une inductance (54S, 60S, 62S) qui forme un circuit résonant avec le condensateur , d'identification (34) lorsque la seconde unité est connectée à la première unité, la fréquence de résonance du circuit résonant étant dépendante de la valeur du condensateur (34).

2. Instrument selon la revendication 1, **caractérisé en ce que** les moyens de détection (50) et le condensateur d'identification (34) forment ensemble un oscillateur dont la fréquence de fonctionnement est dépendante de la valeur du condensateur d'identification (34).

3. Instrument selon la revendication 2, **caractérisé en ce que** l'inductance (54S) comprend un premier enroulement (54S, 62S) d'un transformateur (54, 62), le condensateur d'identification (34) est connecté entre une première paire de contacts sur la seconde unité, et le premier enroulement du transformateur (54S, 62S) est connecté entre une seconde paire de contacts (36B, 36C) sur la première unité, les première et seconde paires de contacts étant situées de telle sorte que les contacts d'une paire engagent les contacts respectifs de l'autre paire lorsque la première unité est connectée à la seconde unité pour former ledit circuit résonant.

4. Instrument selon la revendication 3, **caractérisé en ce que** le transformateur (54, 62) est un transformateur d'isolement et a un second enroulement (54B, 62A) formant une partie de l'oscillateur.

5. Instrument selon la revendication 4, **caractérisé en ce que** le transformateur (54) a un troisième enroulement (54A) couplé à un dispositif oscillant (52) de l'oscillateur et agissant comme un enroulement d'excitation du circuit résonant.

6. Instrument selon la revendication 4, **caractérisé par** un second transformateur (60) ayant un premier enroulement (60A) couplé à un dispositif oscillant (52) de l'oscillateur et agissant comme un enroulement contenant un circuit résonant, et un second enroulement (60S) couplé en série dans l'enroulement d'excitation du circuit résonant, et un second enroulement (60S) couplé en série dans le circuit résonant.

7. Instrument selon l'une quelconque des revendications 3 à 6, **caractérisé en ce que** l'un des contacts respectifs (28B, 36B) de chacune des première et seconde paires sert également de contact pour conduire des courants d'électrochirurgie radiofréquence entre le générateur (10) et l'ensemble électrode (14).

8. Instrument selon l'une quelconque des revendications 2 à 7, **caractérisé en ce que** les moyens de détection (50) comportent un réseau déphaseur (D1, D2, C3, R4) dans une chaîne de réaction de l'oscillateur.

9. Instrument selon la revendication 8, **caractérisé en ce que** le réseau déphaseur (D1, D2, C3, R4) fournit un déphasage de 90°.

10. Instrument selon la revendication 9, **caractérisé en ce que** le réseau déphaseur comprend une paire de diodes de blocage (D1, D2) connectées avec des polarités opposées, et la combinaison en série d'un condensateur (C3) et d'une résistance (R4).

11. Instrument selon une quelconque revendication précédente, **caractérisé en ce que** les moyens de détection (50) sont sensibles à une pluralité de valeurs différentes du condensateur d'identification (34), et **en ce que** les moyens d'ajustement comprennent un contrôleur (48) disposé pour régler la puissance de sortie du générateur suivant un signal de sortie fourni par les moyens de détection (50) et représentatif de la valeur du condensateur d'identification.

12. Instrument selon la revendication 11, **caractérisé en ce que** le contrôleur (48) est utilisable pour ajuster la tension d'alimentation moyenne fournie à un circuit de sortie radiofréquence du générateur en réponse au signal de sortie des moyens de détection (50).

13. Instrument selon la revendication 12, **caractérisé en ce que** le générateur (10) comporte une alimentation en mode commuté (42), et le contrôleur (48) est couplé à l'alimentation (42) et utilisable pour ajuster le cycle de service de la sortie commutée en réponse au signal de sortie des moyens de détection (50).

14. Instrument selon une quelconque revendication précédente, **caractérisé en ce que** la première unité comprend le générateur (10), un connecteur (36A, 36B, 36C), et un câble (20) pour coupler le générateur (10) au connecteur (36A, 36B, 36C), le câble (20) comportant des conducteurs pour coupler le condensateur d'identification (34) aux moyens de détection (50), et **en ce que** la seconde unité est sous la forme d'un ensemble électrode (14) comportant un connecteur qui s'accouple avec le connecteur (36A, 36B, 36C) de la première unité.

15. Instrument selon la revendication 14, **caractérisé en ce que** ledit connecteur (36A, 36B, 36C) de la première unité est intégré dans un manche (12) de l'instrument.

16. Instrument selon une quelconque revendication précédente, **caractérisé en ce que** les moyens d'ajustement (48) comportent un circuit pour régler une limite de tension de sortie du générateur pour vaporisation de tissu, la limite étant déterminée suivant la propriété du composant d'identification.

17. Instrument selon une quelconque revendication précédente, **caractérisé en ce que** les moyens de détection comportent une capacité de référence d'étalonnage et un commutateur pour commuter la capacité de référence à travers l'inductance.

18. Instrument selon la revendication 1, **caractérisé en ce que** les moyens de détection (50) comportent un oscillateur pour exciter une résonance dans la combinaison du condensateur d'identification et de l'inductance lorsque la seconde unité est connectée à la première unité, l'oscillateur ayant une sortie couplée à un transformateur pour fournir une isolation c.c. du condensateur.

19. Instrument selon la revendication 18, **caractérisé en ce que** le condensateur d'identification (24) est couplé à une sortie couplée à un transformateur de l'oscillateur par des première et seconde paires de contacts, une paire de contacts formant une partie de la première unité et l'autre formant une partie de la seconde unité, et **en ce que** l'un (28B, 36B) de chacune des deux paires de contacts sert également pour la conduction de courants électrochirurgicaux radiofréquence entre le générateur (10) et l'ensemble électrode (14), et l'autre contact de chaque paire de contacts est un contact d'identification.

20. Instrument selon la revendication 19, **caractérisé en ce que** chacune desdites première et seconde unités n'a qu'un seul dit contact d'identification.

21. Générateur électrochirurgical pour utilisation avec une pluralité d'ensembles électrodes différents comportant des condensateurs d'identification respectifs ayant différentes valeurs indicatives des caractéristiques des ensembles électrodes, le générateur (10) comportant des moyens de détection (50) sensibles aux valeurs des condensateurs et des moyens (48) pour régler automatiquement la sortie du générateur suivant la caractéristique indiquée d'un ensemble électrode sélectionné connecté au générateur, **caractérisé en ce que** les moyens de détection (50) comportent une inductance (54S, 60S, 62S) disposée pour former un circuit résonant avec le condensateur de chaque ensemble électrode sélectionné, la fréquence de résonance duquel circuit résonant est dépendante de la valeur du condensateur respectif, et les moyens de détection (50) sont utilisables pour produire un signal de sortie électrique pour alimenter les moyens de réglage (48), la nature duquel signal de sortie est dépendante de la fréquence de résonance et, par conséquent, de la valeur du condensateur de l'ensemble électrode sélectionné.

22. Générateur selon la revendication 21, **caractérisé en ce que** les moyens de réglage automatique comprennent un contrôleur (48) disposé pour régler la puissance de sortie du générateur ou une tension de sortie de crête maximale, ou les deux.

23. Générateur selon la revendication 21 ou la revendication 22, **caractérisé en ce que** l'inductance (54S, 60S, 62S) forme une partie d'un oscillateur disposé de telle sorte que sa fréquence d'oscillation est ladite fréquence de résonance.

24. Générateur selon la revendication 23, **caractérisé en ce que** l'oscillateur a une sortie couplée à un transformateur pour fournir une isolation c.c. du condensateur d'identification d'un ensemble électrode sélectionné lorsque connecté au générateur.

25. Générateur selon la revendication 24, **caractérisé en ce que** l'inductance comprend un enroulement (54S, 62S) d'un transformateur d'isolement (54, 62) fournissant la sortie couplée à un transformateur de l'oscillateur.

26. Générateur selon l'une quelconque des revendications 21 à 25, **caractérisé par** une pluralité de contacts (36A, 36B, 36C) pour s'accoupler avec une pluralité respective de contacts associés à un ensemble électrode sélectionné, et **caractérisé en ce que** l'inductance (54S, 60S, 62S) est couplée aux bornes des premier et second contacts (36B, 36C) de ladite pluralité de contacts du générateur, le premier contact constituant un contact d'identification unique (36C) et le second (36B) étant couplé de manière à former une partie du trajet de courant pour délivrer des courants électrochirurgicaux à l'ensemble électrode.

27. Générateur selon la revendication 21, **caractérisé en ce que** les moyens de détection (50) comportent un oscillateur pour exciter une résonance dans le condensateur d'identification de l'ensemble sélectionné et l'inductance (54S, 60S, 62S) formant une partie de là combinaison du générateur et de l'ensemble électrode, l'oscillateur ayant une sortie couplée à un transformateur pour fournir une isolation c.c. du condensateur d'identification.

28. Générateur selon la revendication 27, **caractérisé par** une paire de contacts (36B, 36C) pour s'accoupler avec une paire respective de contacts de chaque ensemble électrode sélectionné, la sortie couplée à un transformateur de l'oscillateur étant couplée à ladite paire de contacts du générateur, dans lequel l'un (36B) de la paire de contacts du générateur sert également pour la conduction de courants électrochirurgicaux radio à l'ensemble électrode, l'autre contact (36C) constituant un contact d'identification.

29. Générateur selon la revendication 28, uniquement **caractérisé par** un seul dit contact d'identification (36C).

30. Procédé d'assemblage et de fonctionnement d'un instrument électrochirurgical comprenant :
la fourniture d'une première unité comportant un générateur électrochirurgical (10) pour générer une puissance radiofréquence, le générateur comportant un circuit de détection (50) ;
la fourniture d'une pluralité de secondes unités comprenant différents ensembles électrodes (14), chacune ayant des moyens d'assemblage détachable à la première unité et chacune comportant un condensateur d'identification (34) ayant une valeur indicative d'une caractéristique de l'ensemble respectif ;
la sélection d'une des secondes unités et l'assembler à la première unité ; et
dans la première unité, la détection de ladite valeur du condensateur et l'ajustement automatiquement de la sortie du générateur en réponse à ladite détection pour s'adapter à la caractéristique de l'ensemble électrode de la seconde unité sélectionnée ;
**caractérisé en ce que** le circuit de détection (50) comporte une inductance (54S, 60S, 62S), **en ce que** le condensateur (34) est disposé pour former un circuit résonant avec l'inductance lorsque la seconde unité est assemblée à la première unité, et **en ce que** la valeur du condensateur est détectée dans la première unité en détectant la fréquence de résonance du circuit résonant.

31. Procédé selon la revendication 30, **caractérisé en ce que** la puissance de sortie nominale du générateur est automatiquement ajustée suivant la valeur détectée du paramètre d'identification.

32. Procédé selon la revendication 30 ou la revendication 31, **caractérisé en ce qu'**une tension limite de vaporisation de tissu est automatiquement ajustée suivant la valeur détectée du paramètre d'identification.

33. Procédé selon la revendication 30, **caractérisé en ce qu'**une caractéristique de la sortie du générateur est ajustée en réponse à la valeur du paramètre du composant d'identification suivant la catégorie de procédure chirurgicale telle que **caractérisée par** la configuration mécanique du connecteur de sortie du générateur auquel l'ensemble électrode est connecté.

34. Jeu de pièces pour assembler un instrument électrochirurgical, comprenant une première unité comportant un générateur électrochirurgical (10) pour générer une tension électrochirurgicale radiofréquence, et une pluralité de secondes unités différentes comportant différents ensembles électrodes (14), chaque seconde unité comportant des moyens (31, 33) d'assemblage à la première unité, dans lequel chaque seconde unité comporte un condensateur d'identification (34) ayant une valeur respective choisie dans une plage de valeurs de capacité et indicative d'une caractéristique de l'ensemble électrode respectif (14), et la première unité comporte des moyens (50) pour détecter la capacité indicatrice lorsque la seconde unité est assemblée à la première unité, et dans lequel le générateur (10) comporte des moyens d'ajustement sensibles aux moyens de détection pour ajuster la sortie du générateur, au moyen desquels la sortie du générateur est automatiquement ajustée pour s'adapter aux différentes caractéristiques des ensembles électrodes des secondes unités lorsqu'elles sont sélectivement assemblées à la première unité, **caractérisé en ce que** les moyens de détection comportent une inductance (54S, 60S, 62S) pour former un circuit résonant avec le condensateur (34) et **en ce que** la sortie du générateur est automatiquement ajustée suivant la fréquence de résonance du circuit résonant.

35. Jeu de pièces selon la revendication 34, **caractérisé en ce que** la première unité comprend le générateur (10), un connecteur (36A, 36B, 36C), et un câble (20) pour coupler le générateur au connecteur, et dans lequel chaque seconde unité comprend un dit ensemble électrode (14) qui lui-même comprend un connecteur pour s'accoupler avec le connecteur (36A, 36B, 36C) de la première unité.
